# EUROPEAN PATENT SPECIFICATION

(11) **EP 2 373 269 B1**
(45) Date of publication and mention of the grant of the patent: **07.05.2014**
(21) Application number: 09835351.9
(22) Date of filing: 21.12.2009
(51) Int. Cl.: A61F 13/02, A61F 13/00, A61F 15/00

(54) **DEVICE FOR FACILITATING APPLICATION OF A PLASTIC FILM TO SKIN**
VORRICHTUNG ZUM AUFTRAGEN EINER KUNSTSTOFFFOLIE AUF DIE HAUT
DISPOSITIF POUR FACILITER L'APPLICATION D'UN FILM PLASTIQUE SUR LA PEAU

(30) Priority: 22.12.2008 SE 0802652
(43) Date of publication of application: 12.10.2011
(73) Proprietor: Mölnlycke Health Care AB, 402 52 Göteborg (SE)
(72) Inventor: SVENSBY, Anna, S--426 77 Västra Frölunda (SE)
(74) Representative: Ekwall, Peter
(86) International application number: PCT/SE2009/051481
(87) International publication number: WO 2010/074640

(56) References cited:
- EP-A1- 0 507 459
- EP-A1- 1 683 502
- WO-A1-98/17216
- WO-A1-2004/073567
- US-A- 4 832 008
- US-A- 6 159 497
- US-A1- 2002 107 466

## Description

### TECHNICAL FIELD

The present invention relates to a device for facilitating application of a plastic film to skin, said plastic film being a component in a wound dressing or other medical article, comprising a stiffening layer removably attached to one side of said film, at least a part of the other side of said film being provided with an adhesive, and a release liner attached to the adhesive.

### BACKGROUND ART

Wound dressings including a thin plastic film having an adhesive coating on one side are relatively widely used and adhesively coated thin plastic films are also used to affix medical articles other than wound dressings to the skin of a person, such as e.g. ostomy bags. A problem with thin adhesively coated plastic films is their tendency to wrinkle and adhere to themselves which make them very difficult to handle. In order to make such film dressings easier to apply to the skin of a person, they are provided with temporary stiffening layer which is removed once the film dressing has been applied. It is very difficult to apply film dressings without such a stiffening layer. Known stiffening layers consist of paper, possibly silicone coated, plastic films or laminates of these materials.

EP 0051935 A2 describes a dressing made of polymeric film and a releasable layer made of paper providing stiffness to the dressing.

The problem with prior art film dressings, of the type as is described in for instance EP 0051935 A2, is that it is difficult to remove the frame without disturbing the applied plastic film. The thin plastic film may be lifted from the skin and wrinkle, which can cause leakage in and out from the wound. It is especially difficult in the corners where the risk is high that the film is lifted from the skin when the frame is drawn around the corner. The wrinkles can also increase the risk for the dressing to be rolled off from the skin if the dressing is exposed to friction during usage. Another drawback with the prior art embodiment as described in said publication is that the frame for removing it from the plastic film has to be drawn all around the film dressing which is time consuming and bring about a rather uncontrolled removal.

An example of a device for applying a thin film provided with a stiffening frame is described in US 5 160315. The frame extends around the whole periphery of the film and is divided in two parts. When the frame is removed, one of said part at time has to be removed. The device is rather time consuming to use as the frame also has to be removed piece by piece around the whole periphery. US 5 160315 suggests kraft papers, polyethylene, polypropylene, polyester and composites of those materials as suitable materials for stiffening layers. The materials suggested provide stiffness to flexible film dressings but do not provide any guidance on choice of materials to solve the problem of conformability and difficulty in application to e.g. uneven body parts.

The more recent W02008/019310 on the other hand presents a complex multi-layer conformable wound dressing having a permanently attached (i.e. not removable) support layer for application of the dressing to e.g. convex surfaces.

Even if existing stiffening layers make it possible to apply film dressings, there is a relatively large risk of failure. Folds can be created in the film when a film dressing is applied to uneven parts of the body of a person, such as heels, hands or elbows. Even detachment of the dressing may occur when it is worn.

The objective of the present invention is to facilitate application of a plastic film to skin and to improve such removable stiffening layers in wound dressings or other medical articles so that the risk of failure or the creating of folds when the dressing or the medical device is applied to skin is eliminated or at least greatly reduced.

### DISCLOSURE OF INVENTION

A device as described in the introduction above is according to the invention characterized in that two grip handles are arranged on the stiffening layer, that the stiffening layer is divided in two regions, which regions are arranged on opposite side of a fictitious line crossing over the film, that one of said grip handles is fastened on one of said regions of the stiffening layer in at least parts of an area adjacent to said line, that the rest of said grip handle is free and extends from said area over said one region of the stiffening layer in a first direction towards one end of the film and that the other grip handle is arranged in the same way on the other of said regions of the stiffening layer with said other grip handle fastened on said other one of said regions of the stiffening layer in at least parts of an area adjacent to said line and with the rest of said other grip handle extending from said area in a second and opposite direction to said first direction and towards an opposite end of the film.

The stiffening layer in such a device can easily be removed in one single step by gripping the two grip handles and draw them in opposite directions. In such a construction the removal of each region of the stiffening frame starts from the centre of the device and ends at a free edge of the thin film. The risk that end edges, such as corners of the thin film are lifted up from the skin has in essential been totally eliminated in a device in accordance with the present invention.

A further advantage with two grip handles that are drawn simultaneously in opposite direction during removal of the stiffening regions is that one eliminates the risk that the fastened film is moved in one direction from its intended position when the stiffening layer is removed. This is important for instance when fastening a cannula.

One embodiment of the invention is characterized in that the stiffening layer is in form of a peripheral frame, which is divided in said two regions.

In one embodiment the invention is further characterized in that the two grip handles have the same form and extension as the peripheral frame.

An embodiment of the invention is characterized in that said two grip handles are symmetrically arranged with the same form and size.

According to an embodiment the invention is characterized in that the grip handles are made of a material which is non-elastic at occurring tensions during removal of said regions of the stiffening layer from the film. It is an advantage if the grip handles are made of a non-elastic material to avoid stretching of the grip handle during the frame removal.

An embodiment of the invention is characterized in that the grip handles are fastened with seams on the top side of said regions of the stiffening layer, which seams are arranged by welding or with the aid of an adhesive.

According to an embodiment the invention is characterized in that the stiffening layer is made of a soft and flexible material with a thickness of 0,5-10 mm. The thickness is measured according to SS-EN: ISO 9073-2.

According to a further modified embodiment the invention is characterized in that said material is stretchable.

It is important that the stiffening layer is so soft and flexible that it can be turned during removal, i.e. so that the two grip handles can be drawn in opposite directions. With such a draw direction the risk is essentially eliminated that the film is lifted from the skin during removal of the stiffening layer. A stretchable stiffening layer has better ability to conform its shape to the shape of an uneven portion of the body of a person than a non-stretchable layer, such as the paper or plastic layers commonly used. The thickness of the stiffening layer is chosen so that the bending strength of the stiffening layer will be in the same range as for such conventional stiffening layers. The stiffness of a stiffening layer according to this embodiment of the invention will still facilitate handling of a thin film of a wound dressing or other medical device before application thereof to the same degree as conventional stiffening layers.

With regards to force distribution during removal it would be ideal if the stiffening layer is so soft that it can be turned 180°. This is however not critical for the function. In order to avoid that the film is lifted up when the stiffening frame is removed it is advantageously that the stiffening layer can be bent to such a degree that it can be withdrawn in a direction parallel to the underlying body surface of the patient, for instance following the curvature of the patient's leg.

The stiffening material may also be elastic.

According to one embodiment the invention is characterized in that said material consists of polymeric foam, such as polyurethane foam or polyolefin foam.

According to another embodiment the invention is characterized in that said material is a non-woven.

According to a further embodiment the invention is characterized in that the stiffening layer comprises several layers of the same or different materials.

According to a further embodiment the invention is characterized in that one material is a layer of non-woven and the other layer is a plastic film.

### BRIEF DESCRIPTION OF DRAWINGS

The invention will in the following be described with reference to the enclosed drawings of which:
Figure 1 shows a film dressing according to prior art.
Figure 2 illustrate the film dressing according to figure 1 during an application phase.
Figure 3 schematically shows in a perspective view a first embodiment of the invention.
Figure 4 schematically shows in a perspective view a second embodiment of the invention.
Figure 5 shows in perspective view the embodiment in accordance with figure 3 during an application phase.

### EMBODIMENT(S) OF THE INVENTION

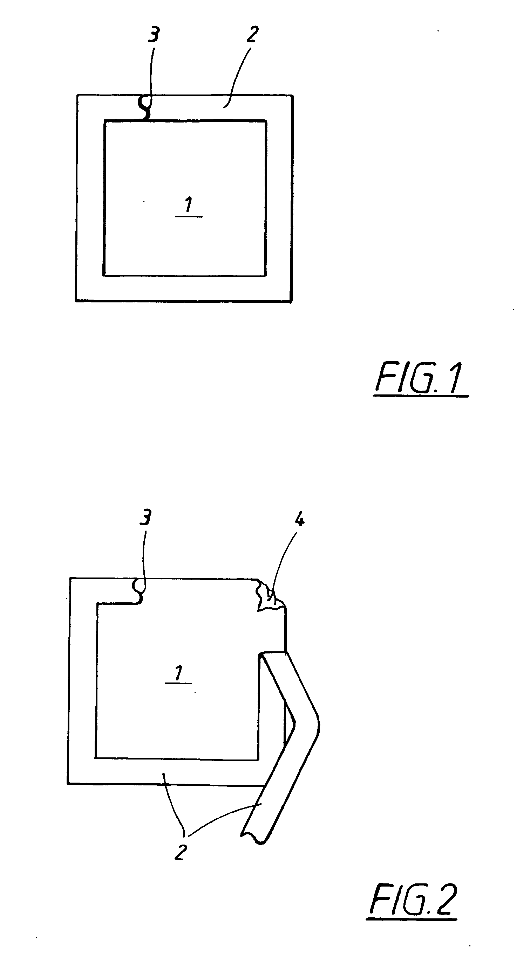

A wound dressing of the type as shown in figure 1 is earlier known and is widely used. The thin plastic film 1 is reinforced with a frame 2 which is removably attached to one side of the film. The other side of the film is provided with an adhesive (not shown) for fastening the film dressing against the skin. The frame is cut along a line 3 as illustrated. The film 1 which often is a polyurethane film is very thin, often as thin as about 10-25 µm. Such a film is difficult to handle and the stiffening frame 2 is used for supporting the film when this is applied on a user. When the film dressing is fastened against the skin the frame is removed. This is illustrated in figure 2. The problem with prior art film dressings is that it is difficult to remove the frame without disturbing the applied plastic film. The thin plastic film may be lifted from the skin and wrinkle, which can cause leakage in and out from the wound. It is especially difficult in the corners as illustrated in figure 2 with the corner 4 which has been lifted from the skin when the frame is drawn around the corner. The wrinkles can also increase the risk for the dressing to be rolled off from the skin if the dressing is exposed to friction during usage. Another drawback with the prior art embodiment shown in figure 1 and 2 is that the frame for removing it from the plastic film has to be drawn all around the film dressing which is time consuming and lead to a rather uncontrolled removal.

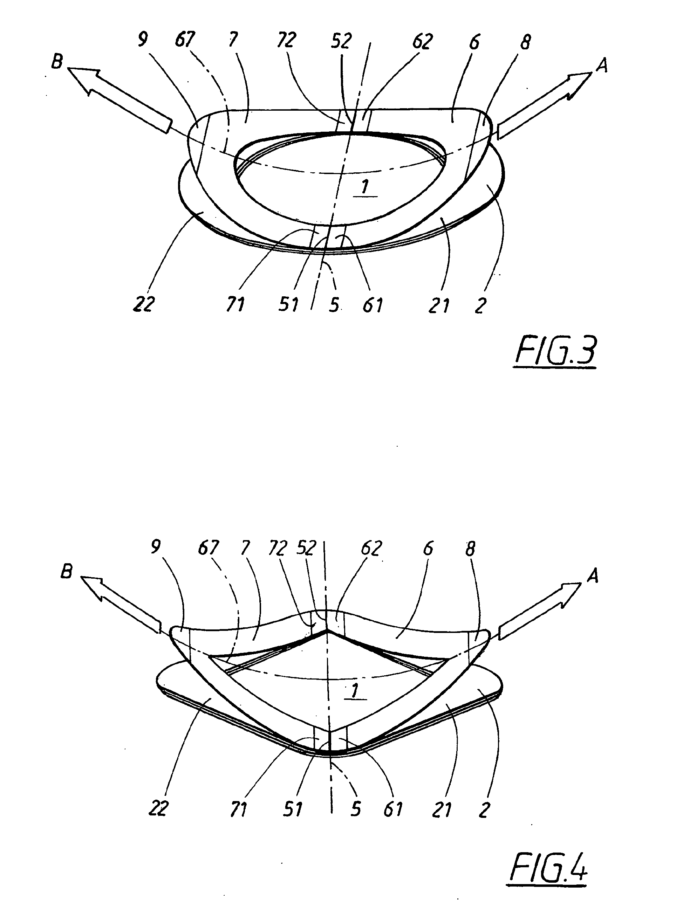

Figure 3 illustrate one embodiment of the improvement according to the present invention. The film and frame in figure 3 have been marked with the same reference number as used on the prior art embodiment. In figure 3 is shown an oval film dressing with a thin oval film 1 and an oval frame 2. The frame 2 is divided in two identical regions 21 and 22 along a fictitious line 5 crossing over the film dressing. The through cuts which divide the frame in two regions have been marked with 51 and 52 in figure 3.

Two grip handles 6, 7 are arranged on the frame 2. One grip handle 6 is fastened on the frame region 21 in two parts 61, 62 adjacent to said through cuts 51 and 52. The other grip handle is arranged in the same way on the other frame region 22 and fastened in the two parts 71, 72 adjacent to said through cuts 51, 52.

The two grip handles 6 and 7 are symmetrically arranged and have the same form and size. In the embodiment shown in figure 3 the size and form of the two grip handles are also the same as the frame regions on which they are fastened. The oval film dressing in accordance with figure 3 is also symmetrical on both sides of the centre line 67 of the grip handles 6 and 7.

When the film dressing has been applied on the skin with the aid of the supporting stiffening frame 2 the grip handles lie flat against the stiffening frame 2. In the shown embodiment the grip handles 6 and 7 are provided with end portions 8 and 9 which may be marked in order to distinguish from the rest of the grip handles in order to facilitate for a user to grip the grip handles. The stiffening frame 2 is removed from the fastened film dressing by pulling the two grip handles 6 and 7 in opposite directions as have been illustrated with the arrows A and B in figure 3. The in opposite directions acting pulling forces act along the centre line 67 which just for illustrative purpose has been shown as perpendicular to said fictitious line 5. The pulling force is divided in the two branches of the respective grip handle and results in a force in each of the branch. The resulting forces acting on the fastening parts 61 and 62 are both of the same size as the resulting forces on the fastening parts 71 and 72. If the user draws in the grip handles in opposite direction of the arrows A and B the force transferred via the fastening parts 61 to the frame region 21 is then acting in an opposite direction to the force transferred via the fastening part 71. The corresponding forces are taken up by the thin plastic film.

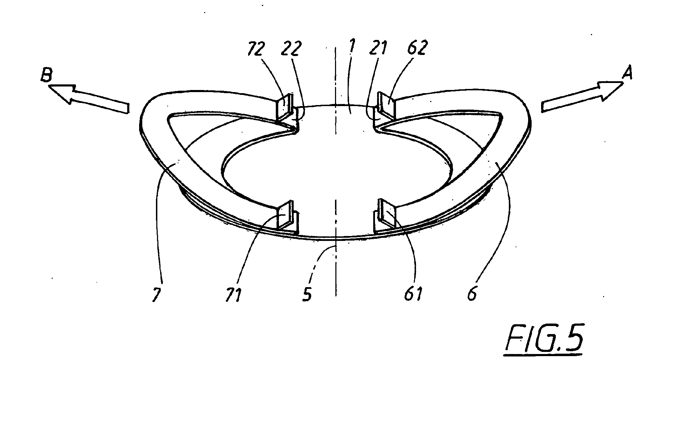

When drawing in the direction of the arrows A and B the frame regions 21 and 22 are withdrawn from the thin film 1 as illustrated in figure 5.

One important advantage with the device in accordance with the invention is that each region of the stiffening frame is removed in a direction that starts from the centre of the film dressing, i.e. where the grip handles are fastened, and ends at a free edge of the film dressing. One therewith avoid that free edges are lifted up when the frame is removed, which is a serious problem with hitherto known film dressings with stiffening frames.

Another advantage with the device in accordance with the invention is that the frame is removed from the film with a movement which is directed obliquely outwards from the border of the film. In earlier known products with a removable frame, the frame is removed with a movement that follows the border of the film. Such earlier known products are illustrated in figure 1 and 2 and the drawback with such products is as mentioned above that the border of the thin film is lifted up when the stiffening layer is removed.

By a movement direction, obliquely outwards, as according to the present invention one avoid that the plastic film is lifted up at the outer border.

It will also be much easier compared with known products to remove the frame from the film and without additional instruction or training. The removal of the frame will also go faster, since the user does not need to draw the frame off the film piece for piece a whole turn around the frame. One also avoid problems at corners, which problems have been illustrated at 4 in figure 2 of the shown prior art embodiment.

In figure 4 is shown a second embodiment of the film dressing in accordance with the invention. Similar parts in embodiment according to figure 2 have been marked with the same reference numbers as corresponding parts in the above described embodiment according to figure 3. The film dressing in figure 4 is square-formed, which means that it comprises sharp corners. In order to avoid corner problems the grip handles are fastened at two opposing corners. With such a construction one avoid that the frame when it is removed has to be drawn around sharp corners. Instead the frame regions 21 and 22 will be drawn from two opposing corners and leave the film from the two other opposing corners.

An adhesive for fastening the thin film in the device in accordance with the invention on the skin is applied over at least a part of the film. The adhesive coating is before use protected in a customary fashion by a release liner (not shown) with low adhesion to the adhesive, for example a plastic film or a silicone coated paper. The release liner is removed before application of the thin film. The function of the stiffening layer is to stiffen up the film in order to facilitate application of the thin film. Without a stiffening layer it would be very difficult to handle the thin film with its coating of adhesive after that the release liner has been removed.

A principal function of the adhesive coating is to attach the film 1 securely to the skin, so that the product remains in place during the normal loadings which film dressings are subjected to. Another function of the adhesive coating is to attach the film 1 tightly to the skin of the patient, so that fluid-borne transport of bacteria in any direction between the skin and the adhesive coating is prevented.

The adhesive in the coating must also be skin friendly and must permit removal of the film dressings without causing damage to the skin.
A silicone gel possesses low surface energy, and it adapts very well to the skin, that is to say it flows out into any unevennesses in the skin and creates a large contact surface between the skin and the soft silicone gel. This large contact surface helps the silicone gel to become attached securely to the skin, in spite of the fact that the strength of the adhesive attachment of the silicone gel to the skin in itself is not so strong. The adhesive strength constitutes a measure of the energy required in order to separate/pull off the adhesive layer from the skin. A contributory factor to the fact that high energy, and thus a high pulling force, is required in order to remove the silicone gel from the skin, in spite of the relatively weak strength of the adhesive attachment, is that a lot of energy is consumed in stretching the soft silicone gel before it releases from the skin. The softer and thicker the layer of silicone gel, the greater the force/energy required to remove the gel from the skin. Examples of suitable silicone gels and methods of measuring softness and adhesion can be found in WO 2006/075950 to which is referred in this respect.

Other adhesives, such a hot-melt or acrylate based adhesives can be used instead of silicone adhesives.

The adhesive need not cover the whole surface of the film and can be applied as a continuous or discontinuous layer.

The stiffening layer 2 may be made of a polymeric foam, for example polyolefin foam or polyurethane foam. Such material has, in contrast to known materials for stiffening layers, the ability to follow the shape of an uneven body portion of a person to which the wound dressing should be applied, such as a heel, a hand, a knee or an elbow. This is due to the fact that polymeric foams are soft and flexible and stretchable and can be applied for example to a heel with much less risk of creating folds than with known film dressings.

The stretchability of the foam shall be less than 2000 kN/m², preferably less than 1000 kN/m², more preferably less than 500 kN/m², and most preferably less than 250 kN/m² measured as the axial nominal stress at 5% elongation. To determine the axial (nominal) stress, the Method ASTM D 882-02 was used to measure the tensile force P. For determination of the material thickness D, the SS-EN: ISO 9073-2 Method A: 1996 was used. The axial nominal stress S was then obtained by the following calculation S=P/(D*W), where W is the sample width.

In contrast to polymeric films and paper, polymeric foam is also compressible, a property that also contributes to reduce the risk for folds to occur when the dressing is applied. By the term "compressible" is primarily meant the reduction of the pore volumes when the foam is subjected to external pressure.

Since the stiffening layer 2 also must perform the task of holding the film layer 1 with its layer of adhesive flat and stretched before and during at least a part of the application procedure without help from the at that time removed release liner, the foam need to be rather thick in order to have a required form stability. The thickness of the foam shall be between 0.5 mm - 10 mm, preferably between 0.75- 7 mm, and most preferably between 1-5 mm. For determination of material thickness, the SS-EN: ISO 9073-2 Method A: 1996 was used.

The stiffening layer 2 can be applied onto the film layer 1 by the application of heat and/or pressure or be glued thereto.

The foam used in the stiffening layer 2 has preferably closed cells. Such a foam presents a larger contact surface to the film 1 than a foam having open cells. Thereby the foam is easier to attach to the film in a controlled manner so that the force needed to remove the stiffening layer from the film after application of the film to skin will have the desired magnitude. The force needed to remove the stiffening layer from the film will also be more even over the whole surface attached to the film 1 when the foam used has closed cells than when the foam used has open cells. A user would therefore feel more comfortable to remove a stiffening layer consisting of foam with closed cells than foam with open cells.

Foam with open cells absorbs liquids. If glue in liquid form is used for attaching a stiffening layer 2 consisting of a foam with open cells to the film 1, it can be hard to determine the amount of glue needed in order to attain a desired removal force. Furthermore, the amount of glue necessary will be larger than if a foam having closed cells were used instead. Thus, although a foam with open cells could be used it is more advantageous to use a foam with closed cells for the stiffening layer since it is easier to control the attachment of the stiffening layer to the film when a foam with closed cells is used.

Examples of suitable foam materials for the present invention are Alveolit® TA 3001 and Alveolit® TEE 3002, both physically cross-linked, closed-cell polyolefin foams, and Alveo-Soft® SAVM200503.00, cross-linked polyolefin soft foam with partly open-cell structure, which all can be obtained from SEKISUI ALVEO AG, Luzern, Switzerland.

Other porous materials than foams can be used in stiffening layers according to the present invention. By "porous material" is in this application meant a material having several small, distributed voids within its volume independent of whether the voids are closed or not. It is believed that the presence of voids within the volume of the material in the stiffening layer contributes to the ability of the stiffening layer to follow the shape of an uneven portion of body of the patient when the dressing is applied. Therefore, for example fibrous materials can be used for stiffening layers according to the present invention. An example of suitable fibrous materials is nonwoven materials having a high porosity.

In order to facilitate the attachment of such materials to the film of the wound dressing or other medical device so that an even and easily pre-determined removal force is obtained for the stiffening layer, such materials can be laminated to a plastic film on the side thereof which is to be turned against the film. The attachment of a plastic film to the film of the dressing is easy to control so that the removal force will be even over the surface of the stiffening layer and suitably high. Such a plastic film for facilitating attachment can also be used when the stiffening layer includes a foam with open cells.

The porosity of foam or other porous materials used in the stiffening layer should preferably be at least 80%, more preferably at least 90% and most preferably at least 95%.

The embodiments described above can of course be modified without leaving the scope of invention. Other medical articles than wound dressings can be provided with a device in accordance with the invention. For example ostomy bags, bandages, incision films, and surgical drapes.

The film dressings can have another shape than the dressing according to figures 3-5, and can be of another type, for example a so called island dressing including a wound pad. Furthermore, other plastic materials than polyurethane, for example polyethylene, polyester, or silicone, can be used as adhesive coated film layer in the wound dressing or other medical article according to the present invention. The invention shall therefore only be limited by the content of the enclosed patent claims.

The shape of the film may for instance be round or oval or have a rectangular shape.

In the embodiments shown in figures 3-5 the frame has through cuts 51, 52 which both follow the fictitious line 5. This may lead to that the film dressing bends along a line formed by the through cuts 51 and 52. This can be avoided by arranging the two through cuts so that they do not follow a common line but instead incline in different directions.

The through cuts are of course not necessarily in form of straight lines. The through cuts on the frame in accordance with the present invention can for instance also be curved as shown in figure 1 and 2.

In the above described embodiments the grip handles are fastened to the frame over the entire parts 61,62 and 71,72. To further secure that the stiffening layer is withdrawn from the thin plastic film starting from the inner edge of the stiffening frame the grip handles can be fastened to the frame just in areas close to the inner edge of the frame in said parts 61,62 and 71,72.

Each grip handle can also be formed of one single strip of a material, which strip is folded to form an U-shaped or V-shaped handle and which both ends are fastened to one of said regions 21,22 of the stiffening layer.

## Claims

1. A device for facilitating application of a plastic film (1) to skin, said plastic film being a component in a wound dressing or other medical article, comprising a stiffening layer (2) removably attached to one side of said film, at least a part of the other side of said film being provided with an adhesive, and a release liner attached to the adhesive, **characterized in that** two grip handles (6,7) are arranged on the stiffening layer (2), that the stiffening layer is divided in two regions (21,22), which regions are arranged on opposite side of a fictitious line (5) crossing over the film, that one (6) of said grip handles is fastened on one (21) of said regions of the stiffening layer (2) in at least parts (61,62) of an area adjacent to said fictitious line, that the rest of said grip handle (6) is free and extends from said area over said one region (21) of the stiffening layer in a first direction towards one end of the film (1) and that the other grip handle (7) is arranged in the same way on the other (22) of said regions of the stiffening layer with said other grip handle (7) fastened on said other one (22) of said regions of the stiffening layer in at least parts (71,72) of an area adjacent to said fictitious line (5) and with the rest of said other grip handle (7) extending from said area in a second and opposite direction to said first direction and towards an opposite end of the film (1).

2. A device in accordance with claim 1, **characterized in that** the stiffening layer (2) is in form of a peripheral frame, which is divided in said two regions (21,22).

3. A device in accordance with claim 2, **characterized in that** the two grip handles (6,7) have the same form and extension as the peripheral frame.

4. A device according to any of the preceding claims, **characterized in that** said two grip handles (6,7) are symmetrically arranged with the same form and size.

5. A device according to any of the preceding claims, **characterized in that** the grip handles (6,7) are made of a material which is non-elastic at occurring tensions during removal of said regions of the stiffening layer (2) from the film (1).

6. A device in accordance with any of the preceding claims, **characterized in that** the grip handles (6,7) are fastened with seams on the top side of said regions of the stiffening layer, which seams are arranged by welding or with the aid of an adhesive.

7. A device in accordance with any of the preceding claims, **characterized in that** the stiffening layer (2) is made of a soft and flexible material with a thickness of 0,5-10 mm.

8. A device in accordance with claim 7, **characterized in that** said material is stretchable.

9. A device in accordance with claim 8, **characterized in that** said material is elastic.

10. A device in accordance with any of claims 7-9, **characterized in, that** said material consists of polymeric foam, such as polyurethane foam or polyolefin foam.

11. A device in accordance with any of claims 7-9, **characterized in that** said material is a non-woven.

12. A device in accordance with any of the claims 1-6 **characterized in that** the stiffening layer (2) comprises several layers of the same or different materials.

13. A device in accordance with claim 12, **characterized in that** one material is a layer of non-woven and the other layer is a plastic film.

## Patentansprüche

1. Vorrichtung zum Auftragen einer Kunststofffolie (1) auf die Haut, wobei die Kunststofffolie ein Bestandteil in einem Wundverband oder einem anderen medizinischen Artikel ist, umfassend eine Versteifungsschicht (2), die auf einer Seite der Folie entfernbar befestigt ist, wobei zumindest ein Teil der anderen Seite der Folie mit einem Klebemittel versehen ist, und eine Trennfolie, die auf dem Klebemittel befestigt ist, **dadurch gekennzeichnet, dass** zwei Griffe (6,7) auf der Versteifungsschicht (2) angeordnet sind, dass die Versteifungsschicht in zwei Bereiche (21,22) aufgeteilt ist, welche Bereiche auf entgegengesetzten Seiten einer die Folie überquerenden fiktiven Linie (5) angeordnet sind, dass einer (6) der Griffe auf einem (21) der Bereiche der Versteifungsschicht (2) zumindest in Teilen (61,62) einer Fläche angrenzend an der fiktiven Linie festgemacht ist, dass der Rest des Griffes (6) frei ist und sich von der Fläche über den einen Bereich (21) der Versteifungsschicht in eine erste Richtung gegen ein Ende der Folie (1) erstreckt, und dass der andere Griff (7) ebenso auf dem anderen (22) der Bereiche der Versteifungsschicht angeordnet ist, indem der andere Griff (7) auf dem anderen (22) der Bereiche der Versteifungsschicht zumindest in Teilen (71,72) einer Fläche angrenzend an der fiktiven Linie (5) festgemacht ist und sich der Rest des anderen Griffs (7) von der Fläche in eine andere und entgegengesetzte Richtung zur ersten Richtung und gegen ein entgegengesetztes Ende der Folie (1) erstreckt.

2. Vorrichtung nach Anspruch 1, **dadurch gekennzeichnet, dass** die Versteifungsschicht (2) in Form eines peripherischen Rahmens ist, der in die zwei Bereiche (21,22) aufgeteilt ist.

3. Vorrichtung nach Anspruch 2, **dadurch gekennzeichnet, dass** die zwei Griffe (6,7) die gleiche Form und Ausweitung als der peripherische Rahmen aufweisen.

4. Vorrichtung nach einem der vorgehenden Ansprüche, **dadurch gekennzeichnet, dass** die zwei Griffe (6,7) mit der gleichen Form und Größe symmetrisch angeordnet sind.

5. Vorrichtung nach einem der vorgehenden Ansprüche, **dadurch gekennzeichnet, dass** die Griffe (6,7) aus einem Material hergestellt sind, das bei auftretenden Spannungen während des Entfernens der Bereiche der Versteifungsschicht (2) von der Folie (1) unelastisch ist.

6. Vorrichtung nach einem der vorgehenden Ansprüche, **dadurch gekennzeichnet, dass** die Griffe (6,7) mit Säumen auf der oberen Seite der Bereiche der Versteifungsschicht festgemacht sind, welche Säume durch Schweißen oder mittels eines Klebemittels angeordnet sind.

7. Vorrichtung nach einem der vorgehenden Ansprüche, **dadurch gekennzeichnet, dass** die Versteifungsschicht (2) aus einem weichen und flexiblen Material mit einer Dicke von 0,5-10 mm hergestellt ist.

8. Vorrichtung nach Anspruch 7, **dadurch gekennzeichnet, dass** das Material dehnbar ist.

9. Vorrichtung nach Anspruch 8, **dadurch gekennzeichnet, dass** das Material elastisch ist.

10. Vorrichtung nach einem der Ansprüche 7-9, **dadurch gekennzeichnet, dass** das Material aus Polymerschaum wie beispielsweise Polyurethanschaum oder Polyolefinschaum besteht.

11. Vorrichtung nach einem der Ansprüche 7-9, **dadurch gekennzeichnet, dass** das Material ein Vliesstoff ist.

12. Vorrichtung nach einem der Ansprüche 1-6, **dadurch gekennzeichnet, dass** die Versteifungsschicht (2) mehrere Schichten von dem gleichen oder verschiedenen Materialien umfasst.

13. Vorrichtung nach Anspruch 12, **dadurch gekennzeichnet, dass** ein Material eine Schicht von Vliesstoff ist und die andere Schicht eine Kunststofffolie ist.

## Revendications

1. Dispositif pour faciliter l'application d'un film plastique (1) à la peau, ledit film plastique étant un composant dans un pansement de plaie ou dans un autre article médical, comprenant une couche raidisseuse (2) fixée de manière amovible à l'un côté dudit film, au moins une partie de l'autre côté dudit film étant pourvue d'un adhésif, et un revêtement de libération fixé à l'adhésif, **caractérisé en ce que** deux poignées de préhension (6,7) sont disposées sur la couche raidisseuse (2), que la couche raidisseuse est divisée en deux régions (21,22) qui sont disposées sur des côtés opposés d'une ligne fictive (5) traversant au-dessus du film, que l'une (6) desdites poignées de préhension est fixée sur l'une (21) desdites régions de la couche raidisseuse (2) dans au moins des parties (61,62) d'une zone adjacente à ladite ligne fictive, que le reste de ladite poignée de préhension (6) est libre et s'étend à partir de ladite zone au-dessus de ladite une région (21) de la couche raidisseuse dans une première direction vers l'une extrémité du film (1), et que l'autre poignée de préhension (7) est disposée de la même manière sur l'autre (22) desdites régions de la couche raidisseuse avec ladite autre poignée de préhension (7) fixée sur ladite autre (22) desdites régions de la couche raidisseuse dans au moins des parties (71,72) d'une zone adjacente à ladite ligne fictive (5) et avec le reste de ladite autre poignée de préhension (7) s'étendant à partir de ladite zone dans une deuxième direction opposée à ladite première direction et vers une extrémité opposée du film (1).

2. Dispositif selon la revendication 1, **caractérisé en ce que** la couche raidisseuse (2) est en forme d'un cadre périphérique qui est divisée en lesdites deux régions (21,22).

3. Dispositif selon la revendication 2, **caractérisé en ce que** les deux poignées de préhension (6,7) présentent la même forme et la même extension que le cadre périphérique.

4. Dispositif selon l'une quelconque des revendications précédentes, **caractérisé en ce que** lesdites deux poignées de préhension (6,7) sont disposées symétriquement avec la même forme et la même taille.

5. Dispositif selon l'une quelconque des revendications précédentes, **caractérisé en ce que** les poignées de préhension (6,7) sont réalisées en un matériau qui est non-élastique à des tensions se produisant au cours du retrait desdites régions de la couche raidisseuse (2) à partir du film (1).

6. Dispositif selon l'une quelconque des revendications précédentes, **caractérisé en ce que** les poignées de préhension (6,7) sont fixées au moyen de coutures sur le côté supérieur desdites régions de la couche raidisseuse, lesdites coutures étant disposées par soudage ou avec l'aide d'un adhésif.

7. Dispositif selon l'une quelconque des revendications précédentes, **caractérisé en ce que** la couche raidisseuse (2) est réalisée en un matériau souple et flexible avec une épaisseur comprise entre 0,5 et 10 mm.

8. Dispositif selon la revendication 7, **caractérisé en ce que** ledit matériau est extensible.

9. Dispositif selon la revendication 8, **caractérisé en ce que** ledit matériau est élastique.

10. Dispositif selon l'une quelconque des revendications 7 à 9, **caractérisé en ce que** ledit matériau est constitué d'une mousse polymère, telle qu'une mousse de polyuréthane ou une mousse de polyoléfine.

11. Dispositif selon l'une quelconque des revendications 7 à 9, **caractérisé en ce que** ledit matériau est un non-tissé.

12. Dispositif selon l'une quelconque des revendications 1 à 6, **caractérisé en ce que** la couche raidisseuse (2) comprend plusieurs couches de matériaux identiques ou différents.

13. Dispositif selon la revendication 12, **caractérisé en ce que** l'un matériau est une couche de non-tissé et l'autre couche est un film de matière plastique.
